# EUROPEAN PATENT SPECIFICATION

(11) **EP 4 210 605 B1**
(45) Date of publication and mention of the grant of the patent: **23.07.2025**
(21) Application number: 20953403.1
(22) Date of filing: 14.09.2020
(51) Int. Cl.: A61B 18/00, A61B 18/14, A61M 1/00, A61B 17/34, A61B 18/12

(54) **SMOKE EVACUATION DEVICE WITH FLUID STORAGE FOR LAPAROSCOPIC SURGERY**
RAUCHABZUGSVORRICHTUNG MIT FLÜSSIGKEITSSPEICHERUNG FÜR LAPAROSKOPISCHE CHIRURGIE
DISPOSITIF D'ÉVACUATION DE FUMÉE AVEC STOCKAGE DE FLUIDE POUR CHIRURGIE LAPAROSCOPIQUE

(43) Date of publication of application: 19.07.2023
(73) Proprietor: BIO-PROTECH INC., Gangwon-do, 26365 (KR)
(72) Inventor: PARK, Ik Ro, Wonju-si, Gangwon-do 26365 (KR)
(74) Representative: Verscht, Thomas Kurt Albert
(86) International application number: PCT/KR2020/012377
(87) International publication number: WO 2022/055001

(56) References cited:
- WO-A1-2019/106547
- CA-C- 2 929 040
- CN-U- 207 307 413
- KR-A- 20160 138 852
- KR-A- 20180 134 660
- US-A- 5 968 032
- US-A1- 2007 289 449
- US-A1- 2013 231 606
- US-A1- 2019 366 251

## Description

### Technical Field

The present invention relates to a smoke evacuation device with fluid storage for laparoscopic surgery and, more particularly, a smoke evacuation device with fluid storage for laparoscopic surgery, the smoke evacuation device with fluid storage being able to keep moisture so that gas produced in the process of laparoscopic surgery can be easily filtered out.

### Background Art

As the optical technology is developed and various laparoscopic devices are rapidly developed, many operations depending on laparotomy in the past have been replaced with laparoscopic surgery. Laparoscopic surgery, which is an operation of minimally opening the stomach of a patient, inserting various surgical instruments including a surgical camera, and performing an operation while observing the inside the abdominal cavity, has an advantage that it causes less complications such as bleeding, infection, adhesion, etc. after an operation, has an effect in terms of beauty because it cuts a small part, and reduces the hospitalization because of less pain and quick recovery after an operation.

According to the procedure of laparoscopic surgery, an operator inserts a needle into a portion around the navel of a patient after the patient is anesthetized, and then injects carbon dioxide into the abdominal cavity to inflate the abdominal cavity. Thereafter, the operator bores a necessary number of port sites at appropriate positions. The operator inserts a camera through a laparoscopic trocar, whereby the image of the inside of the abdominal cavity is shown through a monitor provided in front of the operator and the operator performs a desired operation using appropriately surgical instruments while looking at the monitor.

Meanwhile, when operating devices such as a laser, an electrosurgical unit, an ultrasonic cutting machine in laparoscopic surgery, a lot of noxious gas is produced during the operation. Such gas obstructs the view of an operator and is malodorous, and may contain chemical and pathological noxious particles such as carbon oxide, carbon dioxide, bacteria, and virus. Accordingly, a gas filter apparatus for removing such gas is disposed in the exhaust tube of laparoscopic trocars to secure safety of an operator against such gas. However, since moisture is contained in the gas that is discharged to the gas filter apparatus, the moisture condenses due to the temperature difference between the inside and the outside of the body when the gas is discharged out of the body. When the water permeates into the gas filter apparatus, the performance of the filter is deteriorated.

From CA 2 929 040 C a medical fluid filter system is known comprising a housing having: a liquid trap chamber having a volume; a filter media chamber; a filter media arranged within the filter media chamber; the liquid trap chamber having a liquid trap outlet port in fluid communication with the filter media chamber; the liquid trap outlet port configured and arranged within the liquid trap chamber to inhibit flow of liquid from the liquid trap chamber to the filter media chamber and configured and arranged to allow gas to flow from the liquid trap chamber to the filter media chamber; a filter system inlet passing through the housing for intake of fluid originating from a surgical site; and a filter system outlet passing through the housing for fluid exhaust.

From US 2013/231 606 A1 a surgical gas delivery system is known that includes a device housing supporting a control unit and a filter interface having a seat for receiving a filter cartridge, the filter cartridge having a filter housing defining an interior reservoir, wherein sensors are coupled to the control unit for sensing a level of liquid within the reservoir of the filter cartridge to prevent contamination of the device.

From WO 2019/106 547 A1 a filter is known including (i) a filter body, (ii) a front cap associated with a first end of the filter body and coupled to and receiving smoke from a vacuum hose, (iii) a back cap associated with a second end of the filter body and having a filter exhaust sized and shaped to associate with and communicate suction from a smoke evacuation system, (iv) a compressed carbon reservoir disposed within the filter body between the front cap and the back cap, and (v) a flexible porous barrier disposed on at least a first side of the compressed carbon reservoir.

### Disclosure of Invention

### Solution to Problem

In order to solve the problems in the related art, an objective of the present invention is to provide a smoke evacuation device with fluid storage for laparoscopic surgery, the smoke evacuation device with fluid storage being able to keep moisture so that gas produced in the process of laparoscopic surgery can be filtered out, as defined in independent claim 1. Preferred embodiments are disclosed in the dependent claims.

In order to achieve the objectives of the present invention, a smoke evacuation device with fluid storage for laparoscopic surgery, which is coupled to an exhaust part of a laparoscopic trocar that is inserted into a human body in laparoscopic surgery, includes: a main body having an empty space and having an intake port formed through a first side thereof to be connected with the exhaust part and a discharge port formed through a second side; and a filter disposed at a first side in the main body, in which a first guide pipe through which fluid flowing into the intake port flows to a second side in the main body and a second guide pipe formed along an outer surface of the first guide pipe are disposed in the main body, and when fluid is guided into the main body by the first guide pipe, moisture in the fluid is kept in the main body and gas in the fluid moves toward the filter through a space between the first guide pipe and the second guide pipe, is filtered out, and is then discharged through the discharge port.

The space in the main body may include a first space that communicates with the intake port, a second space that is disposed at a first side of the first space to communicate with the discharge port and in which the filter is disposed, a third space that is positioned between a second side of the first space and the second space, a second guide hole may be formed at the first side of the first space toward the second space, and a first guide hole may be formed at the second side of the first space toward the third space; the first guide pipe may have a first side connected to the intake port and a second side extending through the first guide hole to be positioned in the third space and has a diameter smaller than the first guide hole; and the second guide pipe may have a first side connected to an inner edge of the first guide hole and a second side extending along the outer surface of the first guide pipe to be positioned in the third space.

A first partition may be formed between the first side of the first space and the second space, a second partition may be formed between the second space and the third space, and a third partition may be formed between the third space and the first space; the second guide hole may be formed through the first partition wall and the first guide hole is formed through the third partition; and fluid guided into the third space through the intake port and the first guide pipe from the exhaust part of the laparoscopic trocar may move through a space between the first guide pipe and the second guide pipe and then may be guided into the first space through the first guide pipe, the fluid guided into the first space may move into the second space through the second guide hole, and gas of the fluid guided into the second space may be filtered out by the filter disposed in the second space and then discharged through the discharge port.

The second guide pipe may have: a funnel portion has: a funnel portion connected to the inner edge of the first guide hole and protruding from the first guide hole such that a width thereof decreases as it goes away from the first guide hole; and an outer extension extending from an end of the funnel portion toward an end of the second guide pipe.

An end of the first guide pipe may protrude further than an end of the second guide pipe.

An end of the first guide pipe and an end of the second guide pipe may not be in contact with an inner side of the third space.

The first guide pipe and the second guide pipe may be spaced apart from a bottom side, which faces the ground, of the inner side of the third space.

The first guide pipe and the second guide pipe may be in parallel with the bottom side.

The intake port may be disposed higher than the first guide hole, and the first guide pipe may have an inclined portion having a first side connected to the intake port and a second side inclined downward toward the first guide hole, and an inner extension extending from the second side of the inclined portion and spaced apart from the bottom side.

A diameter of the inclined portion may increase from the first side thereof positioned at the intake port to the second side thereof positioned at the first guide hole.

The smoke evacuation device with fluid storage may further include a door member adjusting an opening area of the discharge port.

An edge part may protrude outward from the main body along an inner edge of the discharge part; an inner edge of the edge part may have a first inner edge, a second inner edge, and a third inner edge sequentially positioned away from the second space; a blocking portion may be formed to close a first side of the first inner edge and an opening portion may be formed to open a second side of the first edge; a pair of guide rails may be formed at both sides in a longitudinal direction of the second inner edge, the blocking portion may be positioned between first sides of the pair of guide rails, and the opening portion may be positioned between second sides of the pair or guide rails; a cover portion may be formed to cover the third inner edge, a slit may be formed in a longitudinal direction of the cover portion, a first side of the slit may be positioned to face the blocking portion, and a second side of the slit may be positioned to face the opening portion; a first side of the door member may have an area being able to close the opening portion and may slide along the guide rails, and a second side of the door member may protrude out of the edge part through the slit; and when the second side of the door member slides along the slit, the first side of the door member may adjust an opening area of the opening portion while moving along the guide rail.

A cover plate may be formed to cover an inner edge of the discharge port; a through-hole may be formed through a first side, which faces the blocking portion, of the cover plate; the first side of the cover plate which faces the blocking portion may be positioned away from the second guide hole further than a second side of the cover plate; and a first side of the filter may be positioned to cover the second guide hole and a second side of the filter may be positioned to face the through-hole.

The intake port and the first guide hole may be positioned to face each other and the first guide hole may have a larger diameter than the intake port.

The second space may include a first internal space that communicates with the second guide hole, a second internal space that communicates with the discharge port, and a third internal space that is positioned between the first internal space and the second internal space, in which a first internal partition may be formed between the first internal space and the second internal space, a second internal partition may be formed between the second internal space and the third internal space, and a third internal partition may be formed between the second internal space and the first internal space; a first internal hole may be formed through the third internal partition and a second internal hole may be formed through the second internal partition; the filter may be disposed in the first internal space; the smoke evacuation device with fluid storage may further include a door member adjusting an opening area of the first internal hole; and gas of fluid moving into the first internal space through the second guide hole may be filtered out by the filter and then may move into the third internal space through the first internal hole, the gas moving into the third internal space may move into the second internal space through the second internal hole, and the gas moving into the second internal space may be discharged to the discharge port.

The door member may have: a rotary shaft mounted on a first side of the third internal partition, a rotary plate formed in a plate shape to cover the first internal hole, having the rotary shaft inserted in a center thereof, and having an opening hole at a first side, and a knob having a first side connected to the rotary shaft and a second side disposed outside the main body through the main body; and the opening hole may be moved to face or not to face the first internal hole by rotation of the knob, whereby the first internal hole may be opened or closed.

The opening hole may be formed in an arch shape along a rotational circumference of the rotary plate such that a width increases from a first side to a second side; and a size opened to the outside of the first internal hole when the first side of the opening hole is positioned to face the first internal hole may be larger than a size opened to the outside of the first internal hole when the second side of the opening hole is positioned to face the first internal hole.

The discharge port may be positioned at an upper portion of the main body and the intake port may be positioned lower than the discharge port, so gas guided into the main body through the intake port may be manually discharged to the discharge port.

Gas discharged to the discharge portion from the inside of the main body may be automatically discharged by a suction device disposed at the discharge port and suctioning fluid.

### Advantageous Effects of Invention

According to the present invention, water produced in the third space cannot flow back into the second guide plate due to condensation of fluid flowing into the third space, so the performance of the filter is not deteriorated and the filter can easily filter gas flowing into the main body.

Further, since the end of the inner extension protrudes further than the end of the outer extension, water of fluid dropping into the third space from the inner extension does not flow back into the outer extension.

Further, since the funnel portion increases in diameter as it goes away from the outer extension, gas guided into the funnel portion through the outer extension is guided into the first space without a bottleneck.

Further, since the cover plate supports a side of the filter, the filter can be formed large regardless of the size of the discharge port.

Further, since the first guide pipe is inclined downward, moisture contained in fluid flowing inside through the intake port easily flows down to the inner extension along the inclined portion.

### Brief Description of Drawings

The above and other objects, features and other advantages of the present invention will be more clearly understood from the following detailed description when taken in conjunction with the accompanying drawings, in which:
FIG. 1 is a view schematically showing the front of a smoke evacuation device with fluid storage for laparoscopic surgery according to a first embodiment of the present invention;
FIG. 2 is a view schematically showing the rear of the smoke evacuation device with fluid storage for laparoscopic surgery according to the first embodiment of the present invention;
FIG. 3 is a view schematically showing the inside of the main body of the smoke evacuation device with fluid storage for laparoscopic surgery according to the first embodiment of the present invention;
FIG. 4 is a view showing an A-A' cross-section of FIG. 3;
FIG. 5 is a view schematically showing the state in which a door member is coupled to an edge part in the smoke evacuation device with fluid storage for laparoscopic surgery according to the first embodiment of the present invention;
FIG. 6 is a view schematically showing the flowing path of fluid that flows into the smoke evacuation device with fluid storage for laparoscopic surgery according to the first embodiment of the present invention;
FIG. 7 is a view schematically showing a smoke evacuation device with fluid storage for laparoscopic surgery according to a second embodiment of the present invention;
FIG. 8 is a view schematically showing a cross-section of the smoke evacuation device with fluid storage for laparoscopic surgery according to the second embodiment of the present invention;
FIG. 9 is a cross-sectional view schematically showing an B-B' cross-section of FIG. 8;
FIG. 10 is a view schematically showing the flowing path of fluid that flows into the smoke evacuation device with fluid storage for laparoscopic surgery according to the second embodiment of the present invention; and
FIG. 11 is a cross-sectional view taken along line C-C' of FIG. 10.

### Mode for the Invention

Hereafter, the smoke evacuation device with fluid storage for laparoscopic surgery according to exemplary embodiments of the present invention are described in detail with reference to the accompanying drawings.

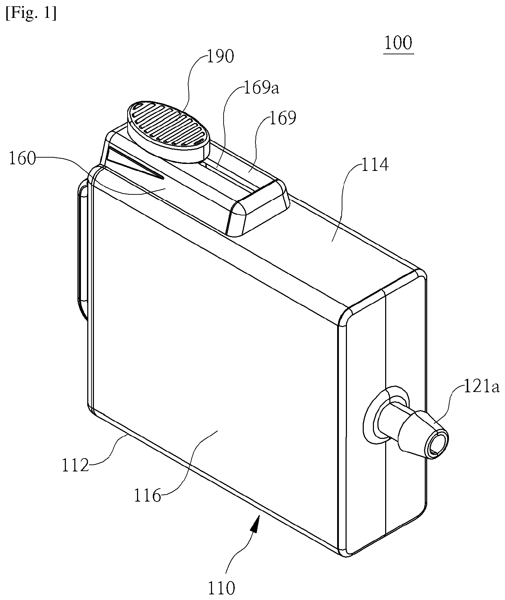

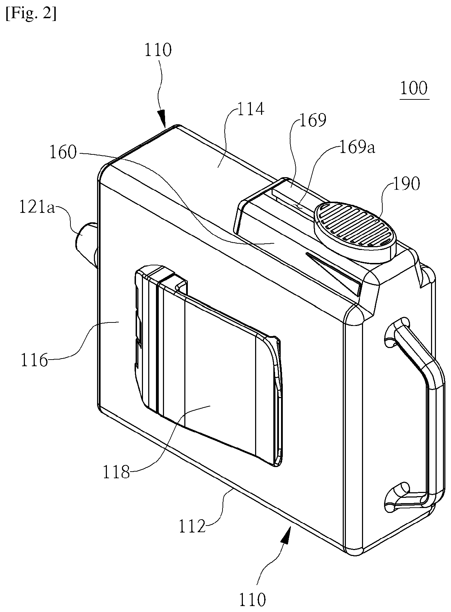

FIG. 1 is a view schematically showing the front of a smoke evacuation device with fluid storage for laparoscopic surgery according to a first embodiment of the present invention and FIG. 2 is a view schematically showing the rear of the smoke evacuation device with fluid storage for laparoscopic surgery according to the first embodiment of the present invention.

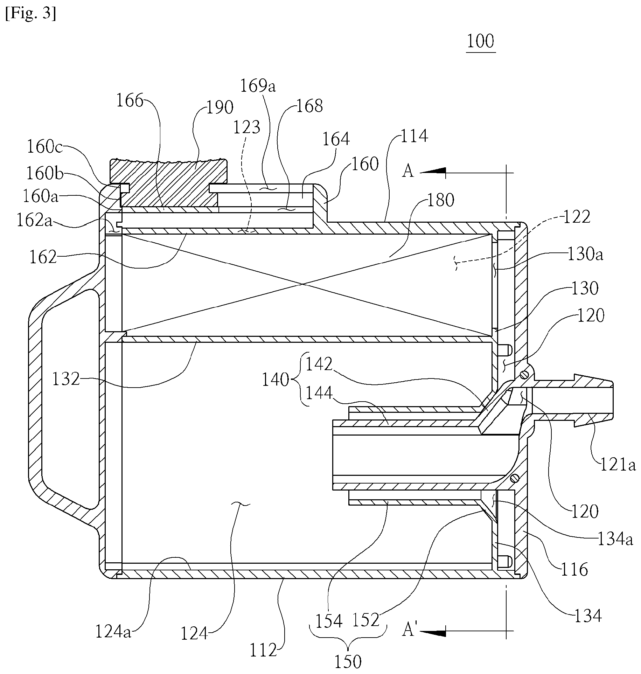

Referring to FIGS. 1 and 2, a smoke evacuation device with fluid storage 100 for laparoscopic surgery according to a first embodiment of the present invention is, for example, coupled to an exhaust part (not shown) of a laparoscopic trocar that is inserted into a human body to filter various gases receiving from the exhaust part of the laparoscopic trocar, and includes a main body 10, a filter 180 (shown in FIG. 3), and a door member 190.

The main body 110 has an empty space therein and is formed in a substantially hexahedral shape. The main body 110 has a top 114, a bottom 112 spaced down apart from the top 114, and a side wall 116 integrally connecting the top 114 and the bottom 112. An intake port 112 that is connected to an exhaust part is formed though a first side of the side wall 116 and a connecting protrusion 121a is formed at the intake port 121 for easy connection to the exhaust part. A coupling portion 118 for fastening the main body 110 at a specific position in an operating room, etc. is disposed on a second side of the side wall 116. A discharge port 123 (shown in FIG. 3) for discharging gas flowing in the main body 110 from the exhaust part of a laparoscopic trocar is formed through the top 114. An edge part 160 is formed along the inner edge of the discharge port 123 and protrudes outward from the main body 110. A cover portion 169 is formed to cover the edge part 160 and a slit 169a is formed in the longitudinal direction of the cover portion 169. The door member 190 adjusts the opening area of the discharge port 123 by sliding in the slit 169a.

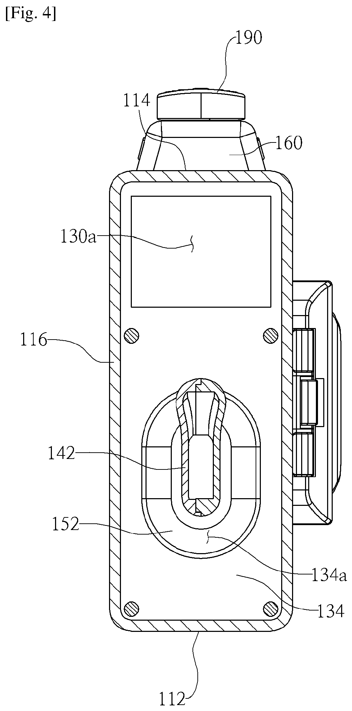

FIG. 3 is a view schematically showing the inside of the main body of the smoke evacuation device with fluid storage for laparoscopic surgery according to the first embodiment of the present invention and FIG. 4 is a view showing an A-A' cross-section of FIG. 3.

Referring to FIGS. 1 to 4, the internal space of the main body 110 includes a first space 120 that communicates with the intake port 121, a second space 122 that communicates with the discharge port 123 and in which a filter 180 is disposed, and a third space 124 that is positioned between the first space 120 and the second space 122. The first, second, and third spaces 120, 122, and 124 are independently separated, and to this end, a first partition 130 is formed between the first space 120 and the second space 122, a second partition 132 is formed between the second space 122 and the third space 124, and a third partition 134 is formed between the third space 124 and the first space 120. A second guide hole 130a is formed through the first partition 130 and a first guide hole 134a is formed through the third partition 134. The intake port 121 has a first guide pipe 140 and a second guide pipe 150 is formed at the first guide hole 134a.

A first side of the first guide pipe 140 is connected to the inner edge of the intake port 121 and a second side thereof is extended and positioned in the third space 124 through the first guide hole 134a. The first guide pipe 140 is smaller in diameter than the first guide hole 134a. The intake portion1 21 is positioned higher than the first guide hole 134a, so the first guide pipe 140 has an inclined portion 142 having a first side connected to the intake port 121 and a second side inclined downward to the first guide hole 134a, and an inner extension 144 extending from the second side of the inclined portion 142 and spaced apart from the bottom 112. Since the first guide pipe 140 is inclined downward, moisture contained in the fluid flowing inside through the intake port easily flows into the inner extension 144 through the inclined portion 142. The diameter of the inclined portion 142 increases toward the second side thereof disposed in the first guide hole 134a from the first side thereof positioned in the intake port 121. Since the inclined portion 142 gradually increases in diameter, gas and moisture easily flow from the inclined portion 142 to the inner extension 144 without a bottleneck.

The second guide pipe 150 has a first side connected to the inner edge of the first guide hole 134a and a second side extended along the outer surface of the first guide pipe 140 and positioned in the third space 124. The second guide pipe 150 is larger in diameter than the first guide pipe 140 such that the first guide pipe 140 is positioned inside the second guide pipe 150. The second guide pipe 150 has a funnel portion 152 connected to the inner edge of the first guide hole 134a and protruding from the first guide hole 134a in a cone shape of which the width decreases as it goes away from the first guide hole 134a, and an outer extension 154 extending from the end of the funnel portion 152 to the end of the second guide pipe 150.

Gas guided from the exhaust part of a laparoscopic trocar into the third space 124 through the intake port 121 and the first guide pipe 140 moves through the space between the first guide pipe 140 and the second guide pipe 150 and is then guided into the first space 120 through the first guide hole 134a. The end of the inner extension 144 protrudes further than the end of the outer extension 154, so there is an effect that moisture dropping into the third space 124 through the inner extension 144 does not flow back into the outer extension 154. Since the water produced in the third space 124 cannot flow back into the second guide pipe 150, the performance of the filter 180 is not influenced by the water in the third space 124, so there is an effect that the filter 180 can easily filter the gas flowing in the main body 110. Since the funnel portion 152 increases in diameter as it goes away from the outer extension 154, there is an effect that gas that is guided to the funnel portion 152 through the outer extension 154 is easily guided into the first space 120 without a bottleneck.

The end of the first guide pipe 140 and the end of the second guide pipe 150 are not in contact with the inner side of the third space 124. Accordingly, there is an effect in terms of structure that the moisture dropping into the third space 124 cannot flow back into the end of the first guide pipe 140 or the end of the second guide pipe 150. Further, according to the present invention, the first guide pipe 140 and the second guide pipe 150 may be spaced apart from the bottom side 124a, which faces the ground, of the inner side of the third space 124. The first guide pipe 140 and the second guide pipe 150 may be in parallel with the bottom side 124a. Accordingly, there is an effect foreign substances such as water dropping down to the bottom side 124a through the first guide pipe 140 and accumulated in the third space 124 cannot flow back into the second guide pipe 150.

The third space 124 has a large volume to be able to keep a sufficient amount of water. However, since the first space 120 connects the third space 124 and the second space 122, the first space 120 may be smaller than the second space 122 and the third space 124.

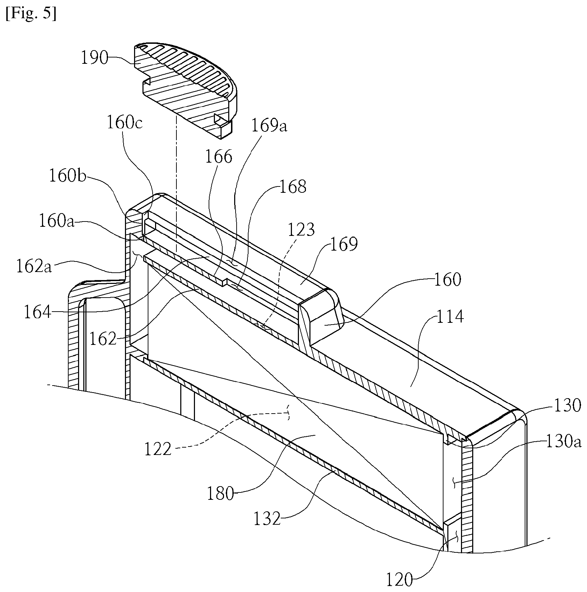

FIG. 5 is a view schematically showing the state in which a door member is coupled to an edge part in the smoke evacuation device with fluid storage for laparoscopic surgery according to the first embodiment of the present invention.

Referring to FIGS. 1 to 5, the filter 180 is disposed in the second space 122. Gas guided into the first space 120 moves into the second space 122 through the second guide hole 130a. The gas guided into the second space 122 is filtered out by the filter 180 disposed in the second space 122 and then discharged through the discharge port 123. The door member 190 is coupled to the edge part 160 to adjust the opening area of the discharge port 123.

The inner edge of the edge part 160 has a first inner edge 160a, a second inner edge 160b, and a third inner edge 160c that are positioned sequentially away from the second space 122. A blocking portion 166 is formed to close a first side of the first inner edge 160a and an opening portion 168 is formed to open a second side of the first inner edge 160a. A pair of recessed guide rails 163 is formed at both sides in the longitudinal direction of the second inner edge 160b, the blocking portion 166 is positioned between first sides of the pair of guide rails 164, and the opening portion 168 is positioned between second sides of the pair or guide rails 164. A cover portion 169 is formed to close the third inner edge 160c, a slit 169a is formed in the longitudinal direction of the cover portion 169 and has a first side facing the blocking portion 166 and a second side facing the opening portion 168.

A first side of the door member 190 has an area being able to close the opening portion 168 and can slide in the guide rails 164 and a second side of the door member 190 protrudes out of the edge part 160 through the slit 169a. When a user holds and slides the second side of the door member 190 along the slit 169a, the first side of the door member 190 moves along the guide rails 164, whereby the opening area of the opening portion 168 is adjusted. That is, when the first side of the door member 190 is positioned to face the opening portion 168, the opening portion 168 is closed, so gas discharged through the discharge port 123 cannot be discharged out of the second space 122. On the contrary, when the first side of the door member 190 is moved to the blocking portion 166 and the opening portion 168 is opened to the slit 169a, the gas discharged through the discharge port 123 is discharged out of the second space 122.

The size of the filter 180 disposed in the second space 122 may be limited, depending on the size of the discharge port 123. That is, gas flows into the filter 180 through a longitudinal first end of the filter 180 and the gas flowing in the filter 180 is filtered out while moving through the filter 180 and is then discharged through a second end of the filter 180. Accordingly, the discharge port 123 should be positioned close to the second end of the filter 180. However, according to the present invention, the door member 190 can move over the discharge port 123, the size of the discharge port 123 should be as large as the movable displacement of the door member 190. This acts as a reason that limits the size of the filter 180.

In order to solve the problem in the present invention, a cover plate 162 is formed to cover the inner edge of the discharge port 123, a through-hole 162a is formed through a first side, which faces the blocking portion 166, of the cover plate 162. Further, the first side of the cover plate 162 which faces the through-hole 162a is positioned away from the second guide hole 130a further than a second side of the cover plate 162. A first end of the filter 180 is positioned to cover the second guide hole 130a and a second end of the filter 180 is positioned to face the through-hole 162a. Since a side of the filter 180 is supported by the cover plate 162, there is an effect that the filter 180 can be formed large regardless of the size of the discharge port 123.

Hereafter, the operation of the smoke evacuation device with fluid storage for laparoscopic surgery according to the first embodiment of the present invention is described.

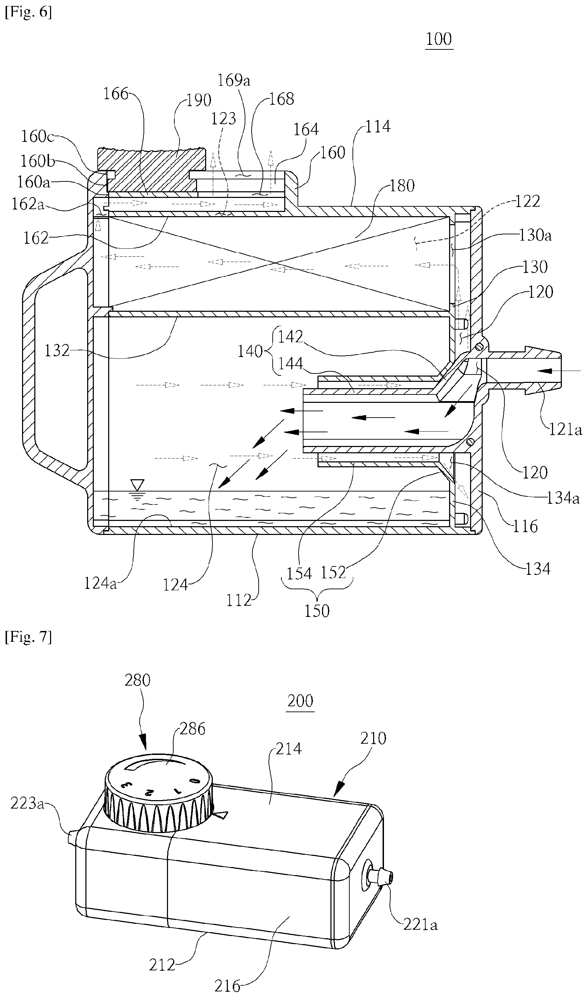

FIG. 6 is a view schematically showing the flowing path of fluid that flows into the smoke evacuation device with fluid storage for laparoscopic surgery according to the first embodiment of the present invention.

Referring to FIGS. 1 to 6, the exhaust part (not shown) of a laparoscopic trocar and the connecting protrusion 121a are connected to each other by a connecting tube (not shown). In this state, a user slides the door member 190 to the blocking portion 166 to open the opening portion 168. Accordingly, fluid flowing into the intake port 121 through the connecting protrusion 121a is guided into the third space 124 through the first guide pipe 140. Moisture contained in the fluid and water produced by condensation due to a temperature difference are kept in the third space 124. Gas moving into the third space 124 moves through the space between the first guide pipe 140 and the second guide pipe 150 and is then guided into the first space 120 through the first guide hole 134a. The gas guided into the first space 120 moves into the second space 122 through the second guide hole 130a. The gas guided into the second space 122 is filtered out by the filter 180 disposed in the second space 122 and then discharged outside sequentially through the through-hole 162a and the opening portion 168.

As described above, since the water produced in the third space 124 due to condensation of the fluid flowing in the third space 124 cannot flow back into the second guide pipe 150, the performance of the filter 180 is not deteriorated, so the filter 180 can easily filter the gas flowing in the main body 110.

FIG. 7 is a view schematically showing a smoke evacuation device with fluid storage for laparoscopic surgery according to a second embodiment of the present invention.

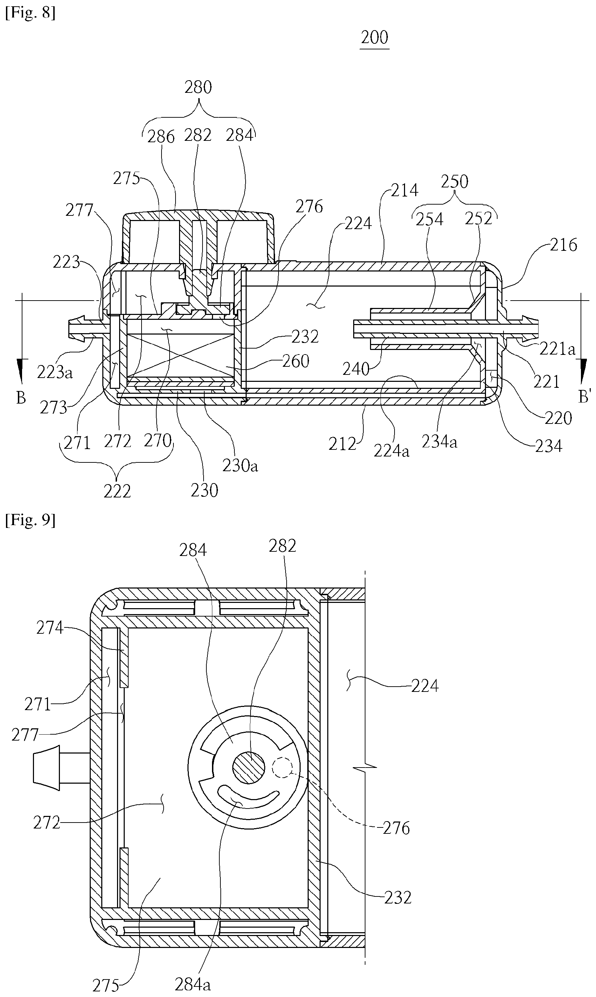

Referring to FIG. 7, a smoke evacuation device with fluid storage 200 for laparoscopic surgery according to a second embodiment of the present invention includes a main body 210, a filter 260 (shown in FIG. 8), and a door member 280.

The main body 210 has an empty space therein and has a top 214, a bottom 212 spaced down apart from the top 214, and a side wall 216 integrally connecting the top 214 and the bottom 212. An intake port 221 for connection with an exhaust part (not shown) of a laparoscopic trocar is formed through a first side of the side wall 216 and a connecting protrusion 221a is formed at the intake port 221. A discharge port 223 for discharging gas flowing in the main body 210 to the outside is formed through a second side of the side wall 216. The door member 280 is rotatably disposed on the top 214 and the amount of gas that is discharged out of the main body 210 depends on the rotation angle of the door member 280.

FIG. 8 is a view schematically showing a cross-section of the smoke evacuation device with fluid storage for laparoscopic surgery according to the second embodiment of the present invention.

Referring to FIGS. 7 to 8, the internal space of the main body 210 includes a first space 220 that communicates with the intake port 221, a second space that communicates with the discharge port 223 and in which the filter 260 is disposed, and a third space 224 that is positioned between the first space 220 and the second space 222. The first, second, and third spaces 220, 222, and 224 are independently separated, and to this end, a first partition 230 is formed between the first space 220 and the second space 222, a second partition 232 is formed between the second space 222 and the third space 224, and a third partition 234 is formed between the third space 224 and the first space 220. A second guide hole 230a is formed through the first partition 230 and a first guide hole 234a is formed through the third partition 234. The first guide hole 232a and the intake port 221 face each other. The first guide hole 234a has a diameter larger than the intake port 221. The intake port 221 has a first guide pipe 240 and a second guide pipe 250 is formed at the first guide hole 234a.

A first side of the first guide pipe 240 is connected to the inner edge of the intake port 221 and a second side thereof is extended and positioned in the third space 224 through the first guide hole 234a. The first guide pipe 240 is smaller in diameter than the first guide hole 234a.

The second guide pipe 250 has a first side connected to the inner edge of the first guide hole 234a and a second side extended along the outer surface of the first guide pipe 240 and positioned in the third space 224. The second guide pipe 250 is larger in diameter than the first guide pipe 240 such that the first guide pipe 240 is positioned inside the second guide pipe 250.

Gas guided from the exhaust part of a laparoscopic trocar into the third space 224 through the intake port 221 and the first guide pipe 240 moves through the space between the first guide pipe 240 and the second guide pipe 250 and is then guided into the first space 220 through the first guide hole 234a.

The end of the first guide pipe 240 and the end of the second guide pipe 250 are not in contact with the inner side of the third space 224. The longitudinal sides of the first guide pipe 240 and the second guide pipe 250 are spaced apart from a bottom side 224a of the third space 224 in parallel with the bottom side 224a, so there is an effect that foreign substances such as water dropping to the bottom side 224a from the first guide pipe 240 and kept in the third space 224 cannot flow back into the second guide pipe 250.

FIG. 9 is a cross-sectional view schematically showing a B-B' cross-section of FIG. 8.

Referring to FIGS. 7 to 9, the second space 222 includes a first internal space 270 that communicates with the second guide hole 230a, a second internal space 271 that communicates with the discharge port 223, and a third internal space 272 that is positioned between the first internal space 270 and the second internal space 271. A first internal partition 273 is formed between the first internal space 270 and the second internal space 271, a second internal partition 274 is formed between the second internal space 271 and the third internal space 272, and a third internal partition 275 is formed between the second internal space 272 and the first internal space 270. A first internal hole 276 is formed through the third internal partition 275 and a second internal hole 277 is formed through the second internal partition 274.

The filter 260 is disposed in the first internal space 270 and filters out gas flowing into the first internal space 270 through the second guide hole 230a. The gas filtered out by the filter 260 moves into the third space 272 through the first internal hole 276. The door member 280 is provided to adjust the opening area of the first internal hole 276.

The door member 280 has a rotary shaft 282 mounted on a first side of the third internal partition 275, a rotary plate 284 formed in a plate shape to cover the first internal hole 276, having the rotary shaft 282 inserted in the center thereof, and having an opening hole 284 at a first side, and a knob 286 having a first side connected to the rotary shaft 282 and a second side disposed outside the main body 210 through the main body 210. The knob 286 is formed to be easily held and rotated by a user. The opening hole 284a is moved to face or not to face the first internal hole 276 by rotation of the knob 286, whereby the first internal hole 276 is opened or closed. That is, when the rotary plate 284 is rotated by rotation of the knob 286 and a first side of the rotary plate 284 where the opening hole 284a is formed is positioned to face the first internal hole 276, the first internal hole 276 is opened by the opening hole 284a. On the contrary, when a second side of the rotary plate 284 where the opening hole 284a is not formed is positioned to face the first internal hole 276, the first internal hole 276 is closed by the second side of the rotary plate 284.

The opening hole 284a is formed in an arch shape along the rotational circumference of the rotary plate 284. The opening hole 284a increases in width from a first side to a second side. The first side of the opening hole 284a is smaller than the first internal hole 275 and the second side of the opening hole 284a is the same in size as the first internal hole 276. Accordingly, when the first side of the opening hole 284a is positioned to face the first internal hole 276, the size opened to the outside of the first internal hole 276 is smaller than the size of the entire first internal hole 276. However, when the second side of the opening hole 284a is positioned to face the first internal hole 276, the size opened to the outside of the first internal hole 276 is relatively large to corresponding to the size of the entire first internal hole 276. Accordingly, the opening area of the first internal hole 276 is adjusted in accordance with the position of the opening hole 284a facing the first internal hole 276.

Hereafter, the operation of the smoke evacuation device with fluid storage for laparoscopic surgery according to the second embodiment of the present invention is described.

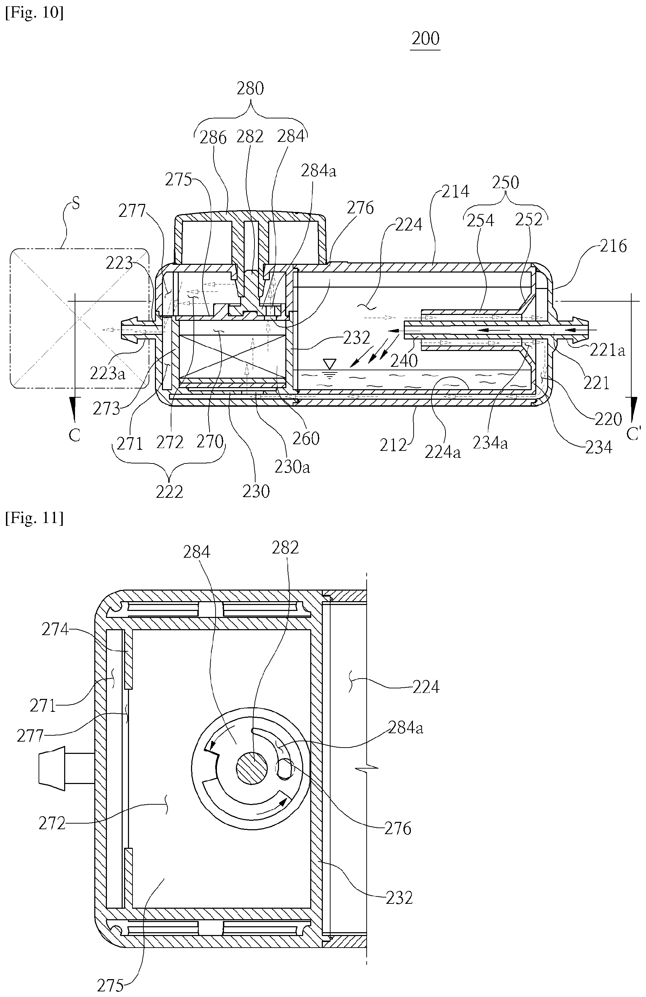

FIG. 10 is a view schematically showing the flowing path of fluid that flows into the smoke evacuation device with fluid storage for laparoscopic surgery according to the second embodiment of the present invention and FIG. 11 is a cross-sectional view taken along line C-C' of FIG. 10.

Referring to FIGS. 10 to 11, the exhaust part (not shown) of a laparoscopic trocar and the connecting protrusion 221a are connected to each other by a connecting tube (not shown). In this state, a user rotates the door member 280 to open the first internal hole 276 to the third internal space 272.

Then, fluid flowing into the intake port 221 through the connecting protrusion 221a is guided into the third space 224 through the first guide pipe 240. Moisture contained in the fluid and water produced by condensation due to a temperature difference are kept in the third space 224. Gas moving into the third space 224 moves through the space between the first guide pipe 240 and the second guide pipe 250 and is then guided into the first space 220 through the first guide hole 234a. The gas of the fluid guided into the first space 220 moves to the first internal space 270 through the second guide hole 230a. The gas moving into the first internal space 270 through the second guide hole 230a is filtered out by the filter 260 and then moves to the third internal space 272 through the first internal hole 276. The gas moving into the third space 272 moves to the second internal space 271 through the second internal hole 277. The gas moving into the second internal space 271 is finally discharged to the discharge port 223. The discharge port 223 has a discharge protrusion 223a and a suction device S that suctions gas is disposed at the discharge protrusion 223a, so the gas discharged to the discharge port 223 from the second internal space 271 is automatically discharged by the suction device S.

The invention may be summarized as follows:
1. A smoke evacuation device with fluid storage for laparoscopic surgery that coupled to an exhaust part of a laparoscopic trocar that is inserted into a human body in laparoscopic surgery, the smoke evacuation device comprising:
   a main body having an empty space and having an intake port formed through a first side thereof to be connected with the exhaust part and a discharge port formed through a second side; and
   a filter disposed at a first side in the main body,
   wherein a first guide pipe through which fluid flowing into the intake port flows to a second side in the main body and a second guide pipe formed along an outer surface of the first guide pipe are disposed in the main body, and when fluid is guided into the main body by the first guide pipe, moisture in the fluid is kept in the main body and gas in the fluid moves toward the filter through a space between the first guide pipe and the second guide pipe, is filtered out, and is then discharged through the discharge port.
2. The smoke evacuation device with fluid storage of item 1, wherein the space in the main body includes a first space that communicates with the intake port, a second space that is disposed at a first side of the first space to communicate with the discharge port and in which the filter is disposed, a third space that is positioned between a second side of the first space and the second space, a second guide hole is formed at the first side of the first space toward the second space, and a first guide hole is formed at the second side of the first space toward the third space;
   the first guide pipe has a first side connected to the intake port and a second side extending through the first guide hole to be positioned in the third space and has a diameter smaller than the first guide hole; and
   the second guide pipe has a first side connected to an inner edge of the first guide hole and a second side extending along the outer surface of the first guide pipe to be positioned in the third space.
3. The smoke evacuation device with fluid storage of item 2, wherein a first partition is formed between the first side of the first space and the second space, a second partition is formed between the second space and the third space, and a third partition is formed between the third space and the first space;
   the second guide hole is formed through the first partition wall and the first guide hole is formed through the third partition; and
   fluid guided into the third space through the intake port and the first guide pipe from the exhaust part of the laparoscopic trocar moves through a space between the first guide pipe and the second guide pipe and is then guided into the first space through the first guide pipe, the fluid guided into the first space moves into the second space through the second guide hole, and gas of the fluid guided into the second space is filtered out by the filter disposed in the second space and is then discharged through the discharge port.
4. The smoke evacuation device with fluid storage of item 2, wherein the second guide pipe comprises:
   a funnel portion connected to the inner edge of the first guide hole and protruding from the first guide hole such that a width thereof decreases as it goes away from the first guide hole; and
   an outer extension extending from an end of the funnel portion toward an end of the second guide pipe.
5. The smoke evacuation device with fluid storage of item 2, wherein an end of the first guide pipe protrudes further than an end of the second guide pipe.
6. The smoke evacuation device with fluid storage of item 2, wherein an end of the first guide pipe and an end of the second guide pipe are not in contact with an inner side of the third space.
7. The smoke evacuation device with fluid storage of item 6, wherein the first guide pipe and the second guide pipe are spaced apart from a bottom side, which faces the ground, of the inner side of the third space.
8. The smoke evacuation device with fluid storage of item 7, wherein the first guide pipe and the second guide pipe are in parallel with the bottom side.
9. The smoke evacuation device with fluid storage of item 7, wherein the intake port is disposed higher than the first guide hole, and
   the first guide pipe has an inclined portion having a first side connected to the intake port and a second side inclined downward toward the first guide hole, and an inner extension extending from the second side of the inclined portion and spaced apart from the bottom side.
10. The smoke evacuation device with fluid storage of item 9, wherein a diameter of the inclined portion increases from the first side thereof positioned at the intake port to the second side thereof positioned at the first guide hole.
11. The smoke evacuation device with fluid storage of item 2, further comprising a door member adjusting an opening area of the discharge port.
12. The smoke evacuation device with fluid storage of item 11, wherein an edge part protrudes outward from the main body along an inner edge of the discharge part;
   an inner edge of the edge part has a first inner edge, a second inner edge, and a third inner edge sequentially positioned away from the second space;
   a blocking portion is formed to close a first side of the first inner edge and an opening portion is formed to open a second side of the first edge;
   a pair of guide rails is formed at both sides in a longitudinal direction of the second inner edge, the blocking portion is positioned between first sides of the pair of guide rails, and the opening portion is positioned between second sides of the pair or guide rails;
   a cover portion is formed to cover the third inner edge, a slit is formed in a longitudinal direction of the cover portion, a first side of the slit is positioned to face the blocking portion, and a second side of the slit is positioned to face the opening portion;
   a first side of the door member has an area being able to close the opening portion and can slide along the guide rails, and a second side of the door member protrudes out of the edge part through the slit; and
   when the second side of the door member slides along the slit, the first side of the door member adjusts an opening area of the opening portion while moving along the guide rail.
13. The smoke evacuation device with fluid storage of item 12, wherein a cover plate is formed to cover an inner edge of the discharge port; a through-hole is formed through a first side, which faces the blocking portion, of the cover plate; the first side of the cover plate which faces the blocking portion is positioned away from the second guide hole further than a second side of the cover plate; and a first side of the filter is positioned to cover the second guide hole and a second side of the filter is positioned to face the through-hole.
14. The smoke evacuation device with fluid storage of item 2, wherein the intake port and the first guide hole are positioned to face each other and the first guide hole has a larger diameter than the intake port.
15. The smoke evacuation device with fluid storage of item 2, wherein the second space includes a first internal space that communicates with the second guide hole, a second internal space that communicates with the discharge port, and a third internal space that is positioned between the first internal space and the second internal space, in which a first internal partition is formed between the first internal space and the second internal space, a second internal partition is formed between the second internal space and the third internal space, and a third internal partition is formed between the second internal space and the first internal space;
   a first internal hole is formed through the third internal partition and a second internal hole is formed through the second internal partition;
   the filter is disposed in the first internal space;
   the smoke evacuation device with fluid storage further includes a door member adjusting an opening area of the first internal hole; and
   gas of fluid moving into the first internal space through the second guide hole is filtered out by the filter and then moves into the third internal space through the first internal hole, the gas moving into the third internal space moves into the second internal space through the second internal hole, and the gas moving into the second internal space is discharged to the discharge port.
16. The smoke evacuation device with fluid storage of item 15, wherein the door member has a rotary shaft mounted on a first side of the third internal partition, a rotary plate formed in a plate shape to cover the first internal hole, having the rotary shaft inserted in a center thereof, and having an opening hole at a first side, and a knob having a first side connected to the rotary shaft and a second side disposed outside the main body through the main body; and
   the opening hole is moved to face or not to face the first internal hole by rotation of the knob, whereby the first internal hole is opened or closed.
17. The smoke evacuation device with fluid storage of item 16, wherein the opening hole is formed in an arch shape along a rotational circumference of the rotary plate such that a width increases from a first side to a second side; and
   a size opened to the outside of the first internal hole when the first side of the opening hole is positioned to face the first internal hole is larger than a size opened to the outside of the first internal hole when the second side of the opening hole is positioned to face the first internal hole.
18. The smoke evacuation device with fluid storage of item 1, wherein the discharge port is positioned at an upper portion of the main body and the intake port is positioned lower than the discharge port, so fluid guided into the main body through the intake port is manually discharged to the discharge port.
19. The smoke evacuation device with fluid storage of item 1, wherein fluid discharged to the discharge portion from the inside of the main body is automatically discharged by a suction device disposed at the discharge port and suctioning fluid.

Although the present invention was described above with reference to the embodiment, the present invention is not limited to the embodiment and it is apparent to those skilled in the art that the present invention may be changed and modified in various ways within the scope of the present invention. Further, the changes and modifications should be construed as being included in the present invention if they belong to the claims.

## Claims

1. A smoke evacuation device with fluid storage (100, 200) for laparoscopic surgery that coupled to an exhaust part of a laparoscopic trocar that is inserted into a human body in laparoscopic surgery, the smoke evacuation device (100, 200) comprising:
a main body (110, 210) having an empty space and having an intake port (121, 221) formed through a first side thereof to be connected with the exhaust part and a discharge port (123, 223) formed through a second side; and
a filter (180, 260) disposed at a first side in the main body (110, 210),
wherein a first guide pipe (140, 240) through which fluid flowing into the intake port (121, 221) flows to a second side in the main body (110, 210) and a second guide pipe (150, 250) formed along an outer surface of the first guide pipe (140, 240) are disposed in the main body (110, 210), and when fluid is guided into the main body (110, 210) by the first guide pipe (140, 240), moisture in the fluid is kept in the main body (110, 210) and gas in the fluid moves toward the filter (180, 260) through a space between the first guide pipe (140, 240) and the second guide pipe (150, 250), is filtered out, and is then discharged through the discharge port (123, 223).

2. The smoke evacuation device with fluid storage (100, 200) of claim 1, wherein the space in the main body (110, 210) includes a first space (120, 220) that communicates with the intake port (121, 221), a second space (122, 222) that is disposed at a first side of the first space (120, 220) to communicate with the discharge port (123, 223) and in which the filter (180, 260) is disposed, a third space (124, 224) that is positioned between a second side of the first space (120, 220) and the second space (122, 222), a second guide hole (130a, 230a) is formed at the first side of the first space (120, 220) toward the second space (122, 222) , and a first guide hole (134a, 234a) is formed at the second side of the first space (120, 220) toward the third space (124, 224);
the first guide pipe (140, 240) has a first side connected to the intake port (121, 221) and a second side extending through the first guide hole (134a, 234a) to be positioned in the third space (124, 224) and has a diameter smaller than the first guide hole (134a, 234a); and
the second guide pipe (150, 250) has a first side connected to an inner edge of the first guide hole (134a, 234a) and a second side extending along the outer surface of the first guide pipe (140, 240) to be positioned in the third space (124, 224).

3. The smoke evacuation device with fluid storage (100, 200) of claim 2, wherein a first partition (130, 230) is formed between the first side of the first space (120, 220) and the second space (122, 222), a second partition (132, 232) is formed between the second space (122, 222) and the third space (124, 224), and a third partition (134, 234) is formed between the third space (124, 224) and the first space (120, 220);
the second guide hole (130a, 230a) is formed through the first partition wall and the first guide hole (134a, 234a) is formed through the third partition (134, 234); and
fluid guided into the third space (124, 224) through the intake port (121, 221), and the first guide pipe (140, 240) from the exhaust part of the laparoscopic trocar moves through a space between the first guide pipe (140, 240) and the second guide pipe (150, 250) and is then guided into the first space (120, 220) through the first guide pipe (140, 150), the fluid guided into the first space (120, 220) moves into the second space (122, 222) through the second guide hole (150, 250), and gas of the fluid guided into the second space (122, 222) is filtered out by the filter (180, 260) disposed in the second space (122, 222) and is then discharged through the discharge port (123, 223).

4. The smoke evacuation device with fluid storage (100, 200) of claim 2, wherein the second guide pipe (150, 250) comprises:
a funnel portion (152) connected to the inner edge of the first guide hole (134a, 234a) and protruding from the first guide hole (134a, 234a) such that a width thereof decreases as it goes away from the first guide hole (134a, 234a); and
an outer extension (154) extending from an end of the funnel portion (152) toward an end of the second guide pipe (150, 250).

5. The smoke evacuation device with fluid storage (100, 200) of claim 2, wherein an end of the first guide pipe (140, 240) protrudes further than an end of the second guide pipe (150, 250).

6. The smoke evacuation device with fluid storage (100, 200) of claim 2, wherein an end of the first guide pipe (140, 240) and an end of the second guide pipe (150, 250) are not in contact with an inner side of the third space (124, 224).

7. The smoke evacuation device with fluid storage (100, 200) of claim 6, wherein the first guide pipe (140, 240) and the second guide pipe (150, 250) are spaced apart from a bottom side (124a, 224a), which faces the ground, of the inner side of the third space (124, 224).

8. The smoke evacuation device with fluid storage (100, 200) of claim 7, wherein the first guide pipe (140, 240) and the second guide pipe (150, 250) are in parallel with the bottom side (124a, 224a).

9. The smoke evacuation device with fluid storage (100, 200) of claim 2, further comprising a door member (190, 280) adjusting an opening area of the discharge port (123, 223).

10. The smoke evacuation device with fluid storage (100, 200) of claim 9, wherein an edge part (160) protrudes outward from the main body (110, 210) along an inner edge of the discharge port;
an inner edge of the edge part (160) has a first inner edge (160a), a second inner edge (160b), and a third inner edge (160c) sequentially positioned away from the second space (122, 222);
a blocking portion (166) is formed to close a first side of the first inner edge (160a) and an opening portion (168) is formed to open a second side of the first inner edge (160a); a pair of guide rails (163) is formed at both sides in a longitudinal direction of the second inner edge (160b), the blocking portion (166) is positioned between first sides of the pair of guide rails (163), and the opening portion (168) is positioned between second sides of the pair or guide rails (164);
a cover portion (169) is formed to cover the third inner edge (160c), a slit (169a) is formed in a longitudinal direction of the cover portion (169), a first side of the slit (169a) is positioned to face the blocking portion (166), and a second side of the slit (169a) is positioned to face the opening portion (168);
a first side of the door member (190, 280) has an area being able to close the opening portion (168) and can slide along the guide rails (164), and a second side of the door member (190, 280) protrudes out of the edge part (160) through the slit (169a); and when the second side of the door member (190, 280) slides along the slit (169a), the first side of the door member (190, 280) adjusts an opening area of the opening portion (168) while moving along the guide rail (164).

11. The smoke evacuation device with fluid storage (100, 200) of claim 10, wherein a cover plate (162) is formed to cover an inner edge of the discharge port (123, 223); a through-hole (162a) is formed through a first side, which faces the blocking portion (166), of the cover plate (162); the first side of the cover plate (162) which faces the blocking portion (166) is positioned away from the second guide hole (130a, 230a) further than a second side of the cover plate (162); and a first side of the filter (180, 260) is positioned to cover the second guide hole (130a, 230a) and a second side of the filter (180, 260) is positioned to face the through-hole (162a).

12. The smoke evacuation device with fluid storage (100, 200) of claim 2, wherein the intake port (121, 221) and the first guide hole (134a, 234a) are positioned to face each other and the first guide hole (134a, 234a) has a larger diameter than the intake port (121, 221).

13. The smoke evacuation device with fluid storage (100, 200) of claim 2, wherein the second space (222) includes a first internal space (270) that communicates with the second guide hole (230a), a second internal space (271) that communicates with the discharge port (223), and a third internal space (272) that is positioned between the first internal space (270) and the second internal space (271), in which a first internal partition (273) is formed between the first internal space (270) and the second internal space (271), a second internal partition (274) is formed between the second internal space (271) and the third internal space (272), and a third internal partition (275) is formed between the second internal space (271) and the first internal space (270);
a first internal hole (276) is formed through the third internal partition (275) and a second internal hole (277) is formed through the second internal partition (274);
the filter (260) is disposed in the first internal space (270);
the smoke evacuation device with fluid storage (200) further includes a door member (280) adjusting an opening area of the first internal hole (275); and
gas of fluid moving into the first internal space (270) through the second guide hole (230a) is filtered out by the filter (260) and then moves into the third internal space (272) through the first internal hole (276), the gas moving into the third internal space (272) moves into the second internal space (271) through the second internal hole (277), and the gas moving into the second internal space (271) is discharged to the discharge port (230).

14. The smoke evacuation device with fluid storage (200) of claim 13, wherein the door member (280) has a rotary shaft (282) mounted on a first side of the third internal partition (276), a rotary plate (284) formed in a plate shape to cover the first internal hole (276), having the rotary shaft (282) inserted in a center thereof, and having an opening hole (284a) at a first side, and a knob (286) having a first side connected to the rotary shaft (282) and a second side disposed outside the main body (210) through the main body (210); and
the opening hole (284a) is moved to face or not to face the first internal hole (276) by rotation of the knob (286), whereby the first internal hole (276) is opened or closed.

15. The smoke evacuation device with fluid storage (200) of claim 14, wherein the opening hole (284a) is formed in an arch shape along a rotational circumference of the rotary plate (284) such that a width increases from a first side to a second side; and
a size opened to the outside of the first internal hole (276) when the first side of the opening hole (284a) is positioned to face the first internal hole (276) is larger than a size opened to the outside of the first internal hole (276) when the second side of the opening hole (284a) is positioned to face the first internal hole (276).

## Patentansprüche

1. Eine Rauchabzugseinrichtung mit Strömungsmittelspeicher (100, 200) für laparoskopische Chirurgie, die mit einem Auslassteil eines laparoskopischen Trokars gekoppelt ist, der bei einer laparoskopischen Chirurgie in einen menschlichen Körper eingesetzt wird, wobei die Rauchabzugseinrichtung (100, 200) folgendes aufweist:
einen Hauptkörper (110, 210) mit einem leeren Raum und mit einem Ansauganschluss (121, 221), der durch eine erste Seite davon gebildet ist, um mit dem Auslassteil verbunden zu sein, und einem Ablassanschluss (123, 223), der durch eine zweite Seite gebildet ist; und
einen Filter (180, 260), der an einer ersten Seite in dem Hauptkörper (110, 210) angeordnet ist,
wobei ein erstes Führungsrohr (140, 240), durch welches Strömungsmittel, das in den Ansauganschluss (121, 221) strömt, zu einer zweiten Seite in dem Hauptkörper (110, 210) strömt, und ein zweites Führungsrohr (150, 250), das entlang einer Außenoberfläche des ersten Führungsrohrs (140, 240) gebildet ist, in dem Hauptkörper (110, 210) angeordnet sind, und wenn Strömungsmittel durch das erste Führungsrohr (140, 240) in den Hauptkörper (110, 210) geführt wird, bleibt Feuchtigkeit in dem Strömungsmittel in dem Hauptkörper (110, 210) und Gas in dem Strömungsmittel bewegt sich durch einen Raum zwischen dem ersten Führungsrohr (140, 240) und dem zweiten Führungsrohr (150, 250) zu dem Filter (180, 260) hin, wird herausgefiltert und dann durch den Ablassanschluss (123, 223) abgelassen.

2. Die Rauchabzugseinrichtung mit Strömungsmittelspeicher (100, 200) nach Anspruch 1, wobei der Raum in dem Hauptkörper (110, 210) einen ersten Raum (120, 220), der mit dem Ansauganschluss (121, 221) in Verbindung steht, einen zweiten Raum (122, 222), der an einer ersten Seite des ersten Raums (120, 220) angeordnet ist, um mit dem Ablassanschluss (123, 223) in Verbindung zu stehen, und in welchem der Filter (180, 260) angeordnet ist, einen dritten Raum (124, 224), der zwischen einer zweiten Seite des ersten Raums (120, 220) und dem zweiten Raum (122, 222) positioniert ist, aufweist, ein zweites Führungsloch (130a, 230a) an der ersten Seite des ersten Raums (120, 220) zu dem zweiten Raum (122, 222) hin gebildet ist, und ein erstes Führungsloch (134a, 234a) an der zweiten Seite des ersten Raums (120, 220) zu dem dritten Raum (124, 224) hin gebildet ist;
das erste Führungsrohr (140, 240) eine erste Seite, die mit dem Ansauganschluss (121, 221) verbunden ist, und eine zweite Seite, die sich durch das erste Führungsloch (134a, 234a) erstreckt, um in dem dritten Raum (124, 224) positioniert zu sein, hat, und einen kleineren Durchmesser als das erste Führungsloch (134a, 234a) hat; und
das zweite Führungsrohr (150, 250) eine erste Seite, die mit einer Innenkante des ersten Führungslochs (134a, 234a) verbunden ist, und eine zweite Seite, die sich entlang der Außenoberfläche des ersten Führungsrohrs (140, 240) erstreckt, um in dem dritten Raum (124, 224) positioniert zu werden, hat.

3. Die Rauchabzugseinrichtung mit Strömungsmittelspeicher (100, 200) nach Anspruch 2, wobei eine erste Trenneinrichtung (130, 230) zwischen der ersten Seite des ersten Raums (120, 220) und dem zweiten Raum (122, 222) gebildet ist, eine zweite Trenneinrichtung (132, 232) zwischen dem zweiten Raum (122, 222) und dem dritten Raum (124, 224) gebildet ist, und eine dritte Trenneinrichtung (134, 234) zwischen dem dritten Raum (124, 224) und dem ersten Raum (120, 220) gebildet ist;
das zweite Führungsloch (130a, 230a) durch die erste Trennwand gebildet ist und das erste Führungsloch (134a, 234a) durch die dritte Trenneinrichtung (134, 234) gebildet ist; und
Strömungsmittel, welches in den dritten Raum (124, 224), und zwar durch den Ansauganschluss (121, 221) und das erste Führungsrohr (140, 240), geführt wird, von dem Auslassteil des laparoskopischen Trokars, bewegt sich durch einen Raum zwischen dem ersten Führungsrohr (140, 240) und dem zweiten Führungsrohr (150, 250) und wird dann durch das erste Führungsrohr (140, 240) in den ersten Raum (120, 220) geführt, das Strömungsmittel, welches in den ersten Raum (120, 220) geführt wird, bewegt sich durch das zweite Führungsloch (150, 250) in den zweiten Raum (122, 222), und Gas des Strömungsmittels, welches in den zweiten Raum (122, 222) geführt wird, wird durch den Filter (180, 260), welcher in dem zweiten Raum (122, 222) angeordnet ist, herausgefiltert (180, 260) und dann durch den Ablassanschluss (123, 223) abgelassen.

4. Die Rauchabzugseinrichtung mit Strömungsmittelspeicher (100, 200) nach Anspruch 2, wobei das zweite Führungsrohr (150, 250) folgendes aufweist:
einen Trichterteil (152), der mit der Innenkante des ersten Führungslochs (134a, 234a) verbunden ist und von dem ersten Führungsloch (134a, 234a) hervorsteht, so dass eine Breite davon mit zunehmender Entfernung von dem ersten Führungsloch (134a, 234a) abnimmt; und
ein äußerer Fortsatz (154), die sich von einem Ende des Trichterteils (152) zu einem Ende des zweiten Führungsrohrs (150, 250) hin erstreckt.

5. Die Rauchabzugseinrichtung mit Strömungsmittelspeicher (100, 200) nach Anspruch 2, wobei ein Ende des ersten Führungsrohrs (140, 240) weiter hervorsteht als ein Ende des zweiten Führungsrohrs (150, 250).

6. Die Rauchabzugseinrichtung mit Strömungsmittelspeicher (100, 200) nach Anspruch 2, wobei ein Ende des ersten Führungsrohrs (140, 240) und ein Ende des zweiten Führungsrohrs (150, 250) nicht mit einer Innenseite des dritten Raums (124, 224) in Kontakt sind.

7. Die Rauchabzugseinrichtung mit Strömungsmittelspeicher (100, 200) nach Anspruch 6, wobei das erste Führungsrohr (140, 240) und das zweite Führungsrohr (150, 250) von einer Unterseite (124a, 224a), welche zu dem Boden hinweist, der Innenseite des dritten Raums (124, 224) beabstandet sind.

8. Die Rauchabzugseinrichtung mit Strömungsmittelspeicher (100, 200) nach Anspruch 7, wobei das erste Führungsrohr (140, 240) und das zweite Führungsrohr (150, 250) parallel zu der Unterseite sind.

9. Die Rauchabzugseinrichtung mit Strömungsmittelspeicher (100, 200) nach Anspruch 2, welche ferner ein Türglied (190, 280) aufweist, das einen Öffnungsbereich des Ablassanschlusses (123, 223) einstellt.

10. Die Rauchabzugseinrichtung mit Strömungsmittelspeicher (100, 200) nach Anspruch 9, wobei ein Kantenteil (160) entlang einer Innenkante des Ablassanschlusses von dem Hauptkörper (110, 210) nach außen hervorsteht;
eine Innenkante des Kantenteils (160) eine erste Innenkante (160a), eine zweite Innenkante (160b) und eine dritte Innenkante (160c), hat, die nacheinander von dem zweiten Raum (122, 222) weg positioniert sind;
ein Sperrteil (166) gebildet ist, um eine erste Seite der ersten Innenkante (160a) zu schließen, und ein Öffnungsteil (168) gebildet ist, um eine zweite Seite der ersten Innenkante (160a) zu öffnen;
ein Paar von Führungsschienen (163) an beiden Seiten in einer Längsrichtung der zweiten Innenkante (160b) gebildet ist, der Sperrteil (166) zwischen ersten Seiten des Paars von Führungsschienen (163) positioniert ist, und der Öffnungsteil (168) zwischen zweiten Seiten des Paars oder Führungsschienen (164) positioniert ist;
ein Abdeckteil (169) gebildet ist, um die dritte Innenkante (160c) abzudecken, ein Schlitz (169a) in einer Längsrichtung des Abdeckteils (169) gebildet ist, eine erste Seite des Schlitzes (169a) positioniert ist, um zu dem Sperrteil (166) hinzuweisen, und eine zweite Seite des Schlitzes (169a) positioniert ist, um zu dem Öffnungsteil (168) hinzuweisen;
eine erste Seite des Türglieds (190, 280) einen Bereich hat, der fähig ist, den Öffnungsteil (168) zu schließen, und entlang der Führungsschienen (164) gleiten kann, und eine zweite Seite des Türglieds (190, 280) durch den Schlitz (169a) aus dem Kantenteil (160) hervorsteht; und
wenn die zweite Seite des Türglieds (190, 280) entlang des Schlitzes (169a) gleitet, stellt die erste Seite des Türglieds (190, 280) einen Öffnungsbereich des Öffnungsteils (168) ein, und zwar während der Bewegung entlang der Führungsschiene (164).

11. Die Rauchabzugseinrichtung mit Strömungsmittelspeicher (100, 200) nach Anspruch 10, wobei eine Abdeckplatte (162) gebildet ist, um eine Innenkante des Ablassanschlusses (123, 223) abzudecken; ein Durchgangsloch (162a) durch eine erste Seite, welche zu dem Sperrteil (166) hinweist, der Abdeckplatte (162) gebildet ist; die erste Seite der Abdeckplatte (162), welche zu dem Sperrteil (166) hinweist, weiter von dem zweiten Führungsloch (130a, 230a) weg positioniert ist als eine zweite Seite der Abdeckplatte (162); und eine erste Seite des Filters (180, 260) positioniert ist, um das zweite Führungsloch (130a, 230a) abzudecken, und eine zweite Seite des Filters (180, 260) positioniert ist, um zu dem Durchgangsloch (162a) hinzuweisen.

12. Die Rauchabzugseinrichtung mit Strömungsmittelspeicher (100, 200) nach Anspruch 2, wobei der Ansauganschluss (121, 221) und das erste Führungsloch (134a, 234a) positioniert sind, um zueinander hinzuweisen, und das erste Führungsloch (134a, 234a) einen größeren Durchmesser als der Ansauganschluss (121, 221) hat.

13. Die Rauchabzugseinrichtung mit Strömungsmittelspeicher (100, 200) nach Anspruch 2, wobei der zweite Raum (222) einen ersten Innenraum (270), der mit dem zweiten Führungsloch (230a) in Verbindung steht, einen zweiten Innenraum (271), der mit dem Ablassanschluss (223) in Verbindung steht, und einen dritten Innenraum (272), der zwischen dem ersten Innenraum (270) und dem zweiten Innenraum (271) positioniert ist, aufweist, in welchem eine erste Innentrenneinrichtung (273) zwischen dem ersten Innenraum (270) und dem zweiten Innenraum (271) gebildet ist, eine zweite Innentrenneinrichtung (274) zwischen dem zweiten Innenraum (271) und dem dritten Innenraum (272) gebildet ist, und eine dritte Innentrenneinrichtung (275) zwischen dem zweiten Innenraum (271) und dem ersten Innenraum (270) gebildet ist;
ein erstes Innenloch (276) durch die dritte Innentrenneinrichtung (275) gebildet ist und ein zweites Innenloch (277) durch die zweite Innentrenneinrichtung (274) gebildet ist;
der Filter (260) in dem ersten Innenraum (270) angeordnet ist;
die Rauchabzugseinrichtung mit Strömungsmittelspeicher (200) ferner ein Türglied (280) aufweist, das einen Öffnungsbereich des ersten Innenlochs (275) einstellt; und
Gas des Strömungsmittels, das sich durch das zweite Führungsloch (230a) in den ersten Innenraum (270) bewegt, durch den Filter (260) herausgefiltert (180, 260) wird und sich dann durch das erste Innenloch (276) in den dritten Innenraum (272) bewegt, das Gas, dass sich in den dritten Innenraum (272) bewegt, bewegt sich durch das zweite Innenloch (277) in den zweiten Innenraum (271), und das Gas, dass sich in den zweiten Innenraum (271) bewegt, wird zu dem Ablassanschluss (230) abgelassen.

14. Die Rauchabzugseinrichtung mit Strömungsmittelspeicher (200) nach Anspruch 13, wobei das Türglied (280) eine Drehwelle (282), die an einer ersten Seite der dritten Innentrenneinrichtung (276) befestigt ist, eine Drehplatte (284) die plattenförmig gebildet ist, um das erste Innenloch (276) abzudecken, und zwar mit der in einer Mitte davon eingesetzten Drehwelle (282) und mit einem Öffnungsloch (284a) an einer ersten Seite, und einen Knopf (286), der eine erste Seite, die mit der Drehwelle (282) verbunden ist, und eine zweite Seite, die außerhalb des Hauptkörpers (210) durch den Hauptkörper (210) angeordnet ist, hat; und
das Öffnungsloch (284a) bewegt wird, um durch die Drehung des Knopfs (286) zu dem ersten Innenloch (276) hinzuweisen oder nicht hinzuweisen, wodurch das erste Innenloch (276) geöffnet oder geschlossen wird.

15. Die Rauchabzugseinrichtung mit Strömungsmittelspeicher (200) nach Anspruch 14, wobei das Öffnungsloch (284a) bogenförmig entlang eines Drehumfangs der Drehplatte (284) gebildet ist, so dass eine Breite von einer ersten Seite zu einer zweiten Seite zunimmt; und
eine zu der Außenseite des ersten Innenlochs (276) hin geöffnete Größe, wenn die erste Seite des Öffnungslochs (284a) positioniert ist, um zu dem ersten Innenloch (276) hinzuweisen, größer als eine zu der Außenseite des ersten Innenlochs (276) hin geöffnete Größe, wenn die zweite Seite des Öffnungslochs (284a) positioniert ist, um dem ersten Innenloch (276) hinzuweisen, ist.

## Revendications

1. Dispositif d'évacuation de fumée avec stockage de fluide (100, 200) pour chirurgie laparoscopique qui est couplé à une partie d'échappement d'un trocart laparoscopique qui est inséré dans un corps humain lors d'une chirurgie laparoscopique, le dispositif d'évacuation de fumée (100, 200) comprenant :
un corps principal (110, 210) ayant un espace vide et ayant un orifice d'admission (121, 221) formé à travers un premier côté de celui-ci pour être relié à la partie d'échappement et un orifice de décharge (123, 223) formé à travers un deuxième côté ; et
un filtre (180, 260) disposé sur un premier côté dans le corps principal (110, 210),
dans lequel un premier tube de guidage (140, 240) à travers lequel un fluide s'écoulant dans l'orifice d'admission (121, 221) s'écoule vers un deuxième côté dans le corps principal (110, 210) et un deuxième tube de guidage (150, 250) formé le long d'une surface extérieure du premier tube de guidage (140, 240) sont disposés dans le corps principal (110, 210), et lorsque le fluide est guidé dans le corps principal (110, 210) par le premier tube de guidage (140, 240), l'humidité dans le fluide est maintenue dans le corps principal (110, 210) et le gaz dans le fluide se déplace vers le filtre (180, 260) à travers un espace entre le premier tube de guidage (140, 240) et le deuxième tube de guidage (150, 250), est filtré, puis est déchargé à travers l'orifice de décharge (123, 223).

2. Dispositif d'évacuation de fumée avec stockage de fluide (100, 200) selon la revendication 1, dans lequel l'espace dans le corps principal (110, 210) comporte un premier espace (120, 220) qui communique avec l'orifice d'admission (121, 221), un deuxième espace (122, 222) qui est disposé sur un premier côté du premier espace (120, 220) pour communiquer avec l'orifice de décharge (123, 223) et dans lequel le filtre (180, 260) est disposé, un troisième espace (124, 224) qui est positionné entre un deuxième côté du premier espace (120, 220) et le deuxième espace (122, 222), un deuxième trou de guidage (130a, 230a) est formé sur le premier côté du premier espace (120, 220) vers le deuxième espace (122, 222), et un premier trou de guidage (134a, 234a) est formé sur le deuxième côté du premier espace (120, 220) vers le troisième espace (124, 224) ;
le premier tube de guidage (140, 240) présente un premier côté relié à l'orifice d'admission (121, 221) et un deuxième côté s'étendant à travers le premier trou de guidage (134a, 234a) pour être positionné dans le troisième espace (124, 224) et a un diamètre plus petit que le premier trou de guidage (134a, 234a) ; et
le deuxième tube de guidage (150, 250) présente un premier côté relié à un bord intérieur du premier trou de guidage (134a, 234a) et un deuxième côté s'étendant le long de la surface extérieure du premier tube de guidage (140, 240) pour être positionné dans le troisième espace (124, 224).

3. Dispositif d'évacuation de fumée avec stockage de fluide (100, 200) selon la revendication 2, dans lequel une première séparation (130, 230) est formée entre le premier côté du premier espace (120, 220) et le deuxième espace (122, 222), une deuxième séparation (132, 232) est formée entre le deuxième espace (122, 222) et le troisième espace (124, 224), et une troisième séparation (134, 234) est formée entre le troisième espace (124, 224) et le premier espace (120, 220) ;
le deuxième trou de guidage (130a, 230a) est formé à travers la première paroi de séparation et le premier trou de guidage (134a, 234a) est formé à travers la troisième séparation (134, 234) ; et
un fluide guidé dans le troisième espace (124, 224) à travers l'orifice d'admission (121, 221), et le premier tube de guidage (140, 240) de la partie d'échappement du trocart laparoscopique se déplace à travers un espace entre le premier tube de guidage (140, 240) et le deuxième tube de guidage (150, 250) et est ensuite guidé dans le premier espace (120, 220) à travers le premier tube de guidage (140, 150), le fluide guidé dans le premier espace (120, 220) se déplace dans le deuxième espace (122, 222) à travers le deuxième trou de guidage (150, 250), et le gaz du fluide guidé dans le deuxième espace (122, 222) est filtré par le filtre (180, 260) disposé dans le deuxième espace (122, 222) et est ensuite déchargé à travers l'orifice de décharge (123, 223).

4. Dispositif d'évacuation de fumée avec stockage de fluide (100, 200) selon la revendication 2, dans lequel le deuxième tube de guidage (150, 250) comprend :
une partie en entonnoir (152) reliée au bord intérieur du premier trou de guidage (134a, 234a) et faisant saillie à partir du premier trou de guidage (134a, 234a) de sorte qu'une largeur de celle-ci diminue à mesure qu'elle s'éloigne du premier trou de guidage (134a, 234a) ; et
une extension extérieure (154) s'étendant depuis une extrémité de la partie en entonnoir (152) vers une extrémité du deuxième tube de guidage (150, 250).

5. Dispositif d'évacuation de fumée avec stockage de fluide (100, 200) selon la revendication 2, dans lequel une extrémité du premier tube de guidage (140, 240) fait davantage saillie qu'une extrémité du deuxième tube de guidage (150, 250).

6. Dispositif d'évacuation de fumée avec stockage de fluide (100, 200) selon la revendication 2, dans lequel une extrémité du premier tube de guidage (140, 240) et une extrémité du deuxième tube de guidage (150, 250) ne sont pas en contact avec un côté intérieur du troisième espace (124, 224).

7. Dispositif d'évacuation de fumée avec stockage de fluide (100, 200) selon la revendication 6, dans lequel le premier tube de guidage (140, 240) et le deuxième tube de guidage (150, 250) sont espacés d'un côté inférieur (124a, 224a), qui est orienté vers le sol, du côté intérieur du troisième espace (124, 224).

8. Dispositif d'évacuation de fumée avec stockage de fluide (100, 200) selon la revendication 7, dans lequel le premier tube de guidage (140, 240) et le deuxième tube de guidage (150, 250) sont parallèles au côté inférieur (124a, 224a).

9. Dispositif d'évacuation de fumée avec stockage de fluide (100, 200) selon la revendication 2, comprenant en outre un organe formant porte (190, 280) ajustant une zone d'ouverture de l'orifice de décharge (123, 223).

10. Dispositif d'évacuation de fumée avec stockage de fluide (100, 200) selon la revendication 9, dans lequel une partie de bord (160) fait saillie vers l'extérieur du corps principal (110, 210) le long d'un bord intérieur de l'orifice de décharge ;
un bord intérieur de la partie de bord (160) présente un premier bord intérieur (160a), un deuxième bord intérieur (160b) et un troisième bord intérieur (160c) positionnés séquentiellement à distance du deuxième espace (122, 222) ;
une partie de blocage (166) est formée pour fermer un premier côté du premier bord intérieur (160a) et une partie d'ouverture (168) est formée pour ouvrir un deuxième côté du premier bord intérieur (160a) ;
une paire de rails de guidage (163) est formée des deux côtés dans une direction longitudinale du deuxième bord intérieur (160b), la partie de blocage (166) est positionnée entre les premiers côtés de la paire de rails de guidage (163) et la partie d'ouverture (168) est positionnée entre les deuxièmes côtés de la paire de rails de guidage (164) ;
une partie de recouvrement (169) est formée pour recouvrir le troisième bord intérieur (160c), une fente (169a) est formée dans une direction longitudinale de la partie de recouvrement (169), un premier côté de la fente (169a) est positionné pour être orienté vers la partie de blocage (166), et un deuxième côté de la fente (169a) est positionné pour être orienté vers la partie d'ouverture (168) ;
un premier côté de l'organe formant porte (190, 280) présente une zone apte à fermer la partie d'ouverture (168) et peut coulisser le long des rails de guidage (164), et un deuxième côté de l'organe formant porte (190, 280) fait saillie à partir de la partie de bord (160) à travers la fente (169a) ; et
lorsque le deuxième côté de l'organe formant porte (190, 280) coulisse le long de la fente (169a), le premier côté de l'organe formant porte (190, 280) ajuste une zone d'ouverture de la partie d'ouverture (168) tout en se déplaçant le long du rail de guidage (164).

11. Dispositif d'évacuation de fumée avec stockage de fluide (100, 200) selon la revendication 10, dans lequel une plaque de recouvrement (162) est formée pour recouvrir un bord intérieur de l'orifice de décharge (123, 223) ; un trou traversant (162a) est formé à travers un premier côté, qui est orienté vers la partie de blocage (166), de la plaque de recouvrement (162) ; le premier côté de la plaque de recouvrement (162) qui est orienté vers la partie de blocage (166) est positionné à distance du deuxième trou de guidage (130a, 230a) plus loin qu'un deuxième côté de la plaque de recouvrement (162) ; et un premier côté du filtre (180, 260) est positionné pour recouvrir le deuxième trou de guidage (130a, 230a) et un deuxième côté du filtre (180, 260) est positionné pour être orienté vers le trou traversant (162a).

12. Dispositif d'évacuation de fumée avec stockage de fluide (100, 200) selon la revendication 2, dans lequel l'orifice d'admission (121, 221) et le premier trou de guidage (134a, 234a) sont positionnés pour être orientés l'un vers l'autre et le premier trou de guidage (134a, 234a) a un diamètre plus grand que celui de l'orifice d'admission (121, 221).

13. Dispositif d'évacuation de fumée avec stockage de fluide (100, 200) selon la revendication 2, dans lequel le deuxième espace (222) comporte un premier espace interne (270) qui communique avec le deuxième trou de guidage (230a), un deuxième espace interne (271) qui communique avec l'orifice de décharge (223), et un troisième espace interne (272) qui est positionné entre le premier espace interne (270) et le deuxième espace interne (271), dans lequel une première séparation interne (273) est formée entre le premier espace interne (270) et le deuxième espace interne (271), une deuxième séparation interne (274) est formée entre le deuxième espace interne (271) et le troisième espace interne (272), et une troisième séparation interne (275) est formée entre le deuxième espace interne (271) et le premier espace interne (270) ;
un premier trou interne (276) est formé à travers la troisième séparation interne (275) et un deuxième trou interne (277) est formé à travers la deuxième séparation interne (274) ;
le filtre (260) est disposé dans le premier espace interne (270) ;
le dispositif d'évacuation de fumée avec stockage de fluide (200) comporte en outre un organe formant porte (280) ajustant une zone d'ouverture du premier trou interne (275) ; et
le gaz du fluide se déplaçant dans le premier espace interne (270) à travers le deuxième trou de guidage (230a) est filtré par le filtre (260) et se déplace ensuite dans le troisième espace interne (272) à travers le premier trou interne (276), le gaz se déplaçant dans le troisième espace interne (272) se déplace dans le deuxième espace interne (271) à travers le deuxième trou interne (277), et le gaz se déplaçant dans le deuxième espace interne (271) est déchargé vers l'orifice de décharge (230).

14. Dispositif d'évacuation de fumée avec stockage de fluide (200) selon la revendication 13, dans lequel l'organe formant porte (280) présente un arbre rotatif (282) monté sur un premier côté de la troisième séparation interne (276), une plaque rotative (284) formée sous une forme de plaque pour recouvrir le premier trou interne (276), ayant l'arbre rotatif (282) inséré au centre de celui-ci, et ayant un trou d'ouverture (284a) sur un premier côté, et un bouton (286) ayant un premier côté relié à l'arbre rotatif (282) et un deuxième côté disposé à l'extérieur du corps principal (210) à travers le corps principal (210) ; et
le trou d'ouverture (284a) est déplacé pour être orienté ou ne pas être orienté vers le premier trou interne (276) par rotation du bouton (286), le premier trou interne (276) étant ainsi ouvert ou fermé.

15. Dispositif d'évacuation de fumée avec stockage de fluide (200) selon la revendication 14, dans lequel le trou d'ouverture (284a) est formé sous une forme d'arc le long d'une circonférence de rotation de la plaque rotative (284) de sorte qu'une largeur augmente d'un premier côté à un deuxième côté ; et
une taille ouverte vers l'extérieur du premier trou interne (276) lorsque le premier côté du trou d'ouverture (284a) est positionné pour être orienté vers le premier trou interne (276) est plus grande qu'une taille ouverte vers l'extérieur du premier trou interne (276) lorsque le deuxième côté du trou d'ouverture (284a) est positionné pour être orienté vers le premier trou interne (276).
